# EUROPEAN PATENT APPLICATION

(11) **EP 3 804 673 A1**
(43) Date of publication of application: **14.04.2021**
(21) Application number: 20201280.3
(22) Date of filing: 12.10.2020
(51) Int. Cl.: A61F 13/00, A61F 13/02

(54) **OPEN WOUND NEGATIVE PRESSURE WOUND THERAPY PROTECTION SYSTEM**

(30) Priority: 11.10.2019 US 201962913918 P
(71) Applicant: Obst, Andrew Thomas, Scandia, MN 55073 (US); Obst, Maryanne Ruth, Scandia, MN 55073 (US); Thomas, Casey James, Scandia, Minnesota 55073 (US)
(72) Inventor: Obst, Andrew Thomas, Scandia, MN 55073 (US); Obst, Maryanne Ruth, Scandia, MN 55073 (US); Thomas, Casey James, Scandia, Minnesota 55073 (US)
(74) Representative: Barker Brettell LLP

(57) **Abstract**

A wound care device (10) for treatment of large wounds with negative pressure that includes an open-cell porous foam layer (46), a skin-safe fluid-permeable contact layer (56) on a bottom surface of the foam layer that is configured to be placed in direct contact with an open wound (31) of a patient, and a top air-sealed layer (47) on an upper surface of foam opposite of the fluid-permeable contact layer. The wound care device (10) can define an outer perimeter (44) along an edge of the foam layer that includes a skin-safe adhesive and is configured to form an air-tight seal in conjunction with the air-sealed layer (47) against a patient's skin.

## Description

### RELATED APPLICATION

The present application claims the benefit of U.S. Provisional Application No. 62/913,918 filed October 11, 2019, which is hereby incorporated herein in its entirety by reference.

### TECHNICAL FIELD

This disclosure relates to medical devices for use in healing large delicate wounds, and in particular, healing of unclosed abdominal wounds, skin graft sites, artificial skin placements, draining wounds which require compressive therapy, and open wounds with enteric fistulas and ostomies.

### BACKGROUND

Surgeons typically attempt full abdominal closure when completing abdominal surgery to include reconnecting the fascia, abdominal muscle layers, and skin. However certain situations arise when a patient's abdominal wall cannot be closed at the time of their initial operation because of trauma, septic abdomen, ischemic bowel, etc. Unfortunately, for many patients, full abdominal closure is impractical or high risk and patients can be left with a large open abdominal wound and exposed bowel.

If the abdominal wall is left open long enough, the body will form a thin layer of granulation tissue over the exposed bowel in an attempt to heal the wound by secondary intent. This healing process can take weeks or months and presents many challenges for the patients and healthcare professionals. These challenges include massive fluid loss, electrolyte imbalances, complex and time-consuming dressing changes, and enteric fistulas when holes in the bowel occur.

Negative Pressure Wound Therapy (NPWT) is often used to facilitate healing of large wounds, however standard NPWT with uncovered foam dressings is not indicated for placement directly on exposed bowel or viscera due to risks of injury to organs and the formation of enteric fistulas.

Two issues involved in treating patients with open abdomens include 1) the mechanical issue of drawing the abdominal wall together to achieve midline closure and 2) the issue that arises when the abdominal wall fails to come together and the underlying viscera is left exposed. Some devices used to address the first issue and provide medial tissue movement and temporary closure of open abdominal wounds include those described in U.S. Patent Publication No. 2014/0221947 A1 to Hunt, U.S. Patent Publication No. 2014/0068914 A1 to Coward, European Patent Publication No. EP 2 538 902 B1 to Lattimore, and International Patent Publication No. WO 2017/019810 A1 to Balasubramaniam, all of which are incorporated herein by reference in their entireties. These devices, however, are typically highly invasive to the body and are used for temporary protection of the bowel and viscera for a relatively short period of time (e.g., 2-10 days). These devices may be utilized between the initial abdominal surgery, while the abdominal cavity is left open, until closure can be achieved. These devices are only designed to facilitate abdominal wall closure and are not designed for use in cases where closure cannot be achieved and the patient is left with an open abdomen with exposed bowel that must heal over an extended period of weeks or months.

Some additional unmet patient needs involve edematous and swollen areas of the body where compression and removal of fluid is the mainstay of treatment. For example, compression with elastic-based materials and fluid absorption is a common treatment for swollen lower extremities with weeping wounds. In these situations, just as with exposed bowel, unprotected NPWT foam dressings are not designed to be placed directly on the skin of the lower extremities.

Traditional NPWT devices are also contraindicated for direct placement over large weeping and swollen wounds involving cutaneous components like swollen lower extremity wounds, scrotal wounds, and any area of the body with wounds and lymphedema. Such NPWT devices are also not appropriate for skin grafts or artificial skin placements.

There exists a need for an improved healing device for large delicate wounds with exposed bowel, skin graft and artificial skin placements, weeping/edematous wounds and swollen extremities with drainage, and open wounds with enteric fistulas and ostomies. Devices are not available that addresses all the issues encountered with these complex wounds: fluid management, wound protection, compression, and ease of use.

### SUMMARY

This disclosure describes embodiments of a Negative Pressure Wound Therapy (NPWT) wound care device for treatment of large wounds with negative pressure in a way that protects organs, skin grafts, fistulas, underlying tissues, and other delicate structures. The disclosed devices are designed to be easy to apply to the surface of the body by bedside nurses or home healthcare providers without specialized equipment or training. Such devices are also uniquely designed to prevent furthering complications and designed to protect the delicate wound surface and the intact skin around a wound.

In some embodiments, the disclosure describes a NPWT wound care device that includes an open-cell porous foam layer, a skin-safe fluid-permeable contact layer on a bottom surface of the foam layer that is configured to be placed in direct contact with an open wound of a patient, and a top air-sealed layer on an upper surface of the foam opposite of the fluid-permeable contact layer. The wound care device defines an outer perimeter along the edge of the foam layer that forms an air-tight seal with the top air-sealed layer. A skin-safe adhesive is placed along the outer perimeter such that the adhesive may be used to bond the wound care device to a patient's skin and form an air-seal over the foam to both enclosed and seal the foam to the patient, allowing a negative pressure (e.g., vacuum or near vacuum) to be applied to the foam, causing the foam to collapse and form a protective layer over an open wound in the patient's skin. Negative pressure in combination with the adhesive materials on the perimeter of the foam form a barrier around the wound opening in the body of the patient and allow for wound drainage to be drawn through the fluid permeable layer and the foam and into an NPWT pump coupled to the wound care device. The skin-safe contact layer on the base of the device allows the device to be placed on both open wounds, including delicate tissue or viscera, and intact skin. The skin and tissue around a wound will be protected from abrasion by the fluid permeable and porous foam layers and receive protected negative pressure wound therapy to reduce edema and swelling incidental to the wound.

In some embodiments the disclosed wound care devices may include channels, cross channels, or the like (e.g., discernible airgaps) in the porous foam layer. The open channels may help to facilitate placement around cylindrical body surfaces and limbs. In this way, the channels enable bending and flexibility along one or more axes while also allowing negative pressure communication throughout the entire device. Additionally, or alternatively, the shape and design of the channels may help direct the lateral (e.g., in-plane) movement of the foam layer during the negative pressure application to help constrict the surface area of the wound care device and help to at least partially close the open wound.

In some embodiments, the foam layer may include channels and cross channels that help facilitate placement on uneven body surfaces. The channels and cross channels may be designed in a way that enables bending and flexibility along at least two axes while also allowing negative pressure communication throughout the entire device.

In some embodiments, the wound care device may be wrapped circumferentially around a limb or the body such that the device at least partially overlaps with itself. In some such examples, the device may attach to itself by fastening the adhesive exterior perimeter over the air-sealed layer in a spiral fashion. Negative pressure wound therapy is then supplied to the foam to compress the overlapped device around the patient's limb or body to help treat circumferential type injuries.

In some embodiments, the disclosed wound care device may be selectively cut to size (e.g., trimmable) to fit the wound. After cutting to size, the outer perimeter of the foam layer may be sealed to the skin with using skin-safe adhesive materials to create the airtight seal. Additionally, or alternatively, the device may be self-sealing such that the perimeters of the wound care device may be sealed prior to or after the device has been cut to size.

In some embodiments, a fistula and ostomy drainage isolator may be embedded in the foam layer for use in open wounds with a fistula, ostomy, or other draining wounds. An opening through the layers of the wound care device and drainage isolator may allow intestinal and other bodily fluid effluent to pass through the device and away from the patient's body for collection into an ostomy pouch-type appliance while maintaining protected negative pressure to the remainder of the wound. In some such examples, the wound care device may be selectively puncturable to allow placement of a fistula and ostomy drainage isolator at any location in the device.

The above summary is not intended to describe each illustrated embodiment or every implementation of the subject matter hereof. The figures and the detailed description that follow more particularly exemplify various embodiments.

### BRIEF DESCRIPTION OF THE DRAWINGS

Subject matter hereof may be more completely understood in consideration of the following detailed description of various embodiments in connection with the accompanying figures, in which:
FIG. 1 is a front view of a patient with an open abdominal wound, exposed bowel, and an enteric fistula.
FIG. 2A is a cross-sectional side view of a wound care device, according to embodiments described herein.
FIG. 2B is an aerial view of the device shown in FIG. 2A.
FIG. 3A is a cross-sectional side view of another example device with channels in the foam layer, according to embodiments described herein.
FIG. 3B is an aerial view of the device shown in FIG. 3A.
FIG. 3C is a cross-sectional side view of the device shown in FIG. 3A in an example employment over a wound.
FIG. 3D is a cross-sectional side view of the device shown in FIG. 3A in an example employment around a circumferential wound.
FIG. 4A is a cross-sectional side view of another example device with channels and cross channels in the foam layer and a fistula and ostomy drainage isolator, according to embodiments described herein.
FIG. 4B is an aerial view of the device shown in FIG. 4A.
FIG. 5A is a cross-sectional side view of another example device with a rounded shape, a negative pressure manifold connector, and shaped channels in the foam layer according to embodiments described herein.
FIG. 5B is an aerial view of the device shown in FIG. 5A.
FIG. 6 is a schematic view of an example medical kit containing a wound care device and subcomponent options, according to embodiments described herein.
FIGS. 7 and 8 are cross-sectional side views of additional example wound care devices according to embodiments described herein.

While various embodiments are amenable to various modifications and alternative forms, the embodiments have been shown by way of example in the drawings and will be described in further detail below. It should be understood, however, that the intention is not to limit the claimed inventions to the particular embodiments described. On the contrary, the intention is to cover all modifications, equivalents, and alternatives falling within the spirit and scope of the subject matter as defined by the claims.

In the following description, reference is made to the accompanying drawings that form a part hereof and in which are shown by way of illustration several specific embodiments. The following reference numbers are used throughout the drawings:

| | |
|---|---|
| 10 | Negative Pressure Wound Therapy (NPWT) wound care device |
| 30 | patient skin |
| 31 | wound or injured skin |
| 32 | enteric fistula, ostomy, or other body opening |
| 34 | intestinal and other bodily fluid effluent |
| 36 | opening in abdominal wall |
| 38 | exposed bowel |
| 39 | patient body |
| 40 | negative pressure pump with pressure P2 less than atmospheric pressure P1 |
| 42 | negative pressure air tube |
| 43 | manifold channel in negative pressure connector |
| 44 | skin-safe adhesive exterior perimeter of NPWT protection device |
| 46 | reticulated open cell foam / porous foam |
| 47 | film or other air impermeable material on top surface of reticulated open cell foam |
| 48 | negative pressure connector |
| 49 | negative pressure connector |
| 50 | channel in reticulated open cell foam |
| 52 | cross-channel in reticulated open cell foam |
| 53 | shaped channel in reticulated open cell foam |
| 54 | reticulated open cell foam bridge for negative pressure communication |
| 56 | fluid-permeable non-adherent skin contact layer |
| 58 | open pathway in the device configured to fit around enteric fistula, ostomy, or other body opening |
| 60 | fluid reservoir |
| 62 | fluid tube |
| 64 | saline solution or antimicrobial wound soak fluid |
| 70 | fistula and ostomy drainage isolator |
| 73 | foam and contact layer subcomponent |
| 75 | negative pressure attachment subcomponent |
| 80 | unified porous contact layer |
| 82 | unified porous layer |
| 84 | self-sealing perimeter region |
| 6 | air impermeable seal |
| 100 | medical kit |

### DETAILED DESCRIPTION OF THE DRAWINGS

The present disclosure describes embodiments of a device for treatment of large wounds with Negative Pressure Wound Therapy (NPWT) in a way that protects bowel, skin graft sites, fistulas and other delicate structures. The device is designed to be easy to apply to the surface of the body by bedside nurses or home healthcare providers without specialized equipment or training. The device may also be designed to be cut to size or overlapped on itself as needed to fit specific patient wound shapes and sizes.

Unless otherwise indicated, all numbers expressing feature sizes, amounts, ranges, and physical properties are to be understood as being modified by the term "about." Accordingly, unless indicated to the contrary, the numerical parameters set forth are approximations that can vary depending upon the desired properties sought to be obtained by those skilled in the art utilizing the teachings disclosed herein.

As used herein, the singular forms "a", "an", and "the" encompass embodiments having plural referents, unless the content clearly dictates otherwise. As used in this specification and the appended claims, the term "or" is generally employed in its sense including "and/or" unless the content clearly dictates otherwise.

The terms "top," "upper," "bottom," or "lower" are used to refer to the relative position of two or more components within the device or a patient's skin.

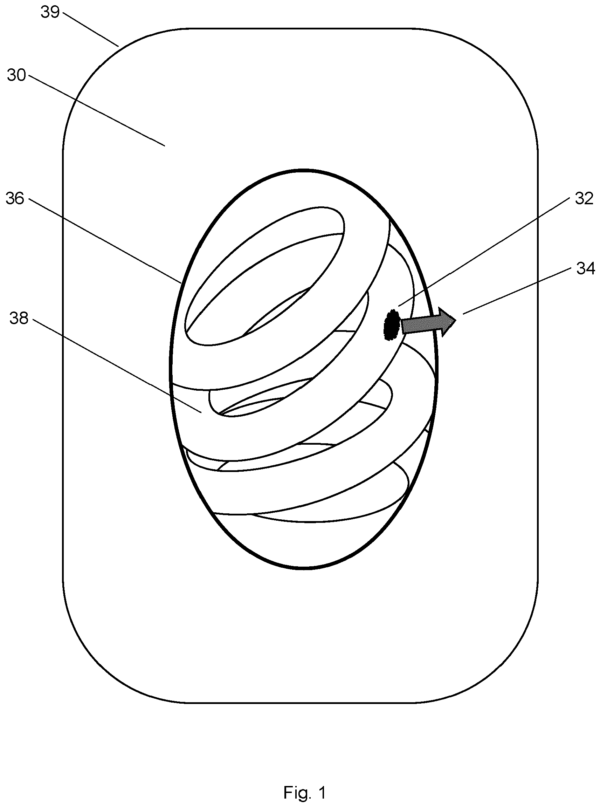

FIG. 1 depicts the front view of a patient's body 39 with an open abdominal wound 36 and exposed bowel 38. Open abdominal wounds typically require weeks or months to heal by secondary intent with granulation tissue, during which time enteric fistulas 32 can form. Managing the drainage of intestinal effluent (represented by arrow 34) from a high output enteric fistula 32 can be a difficult challenge for clinicians and patients.

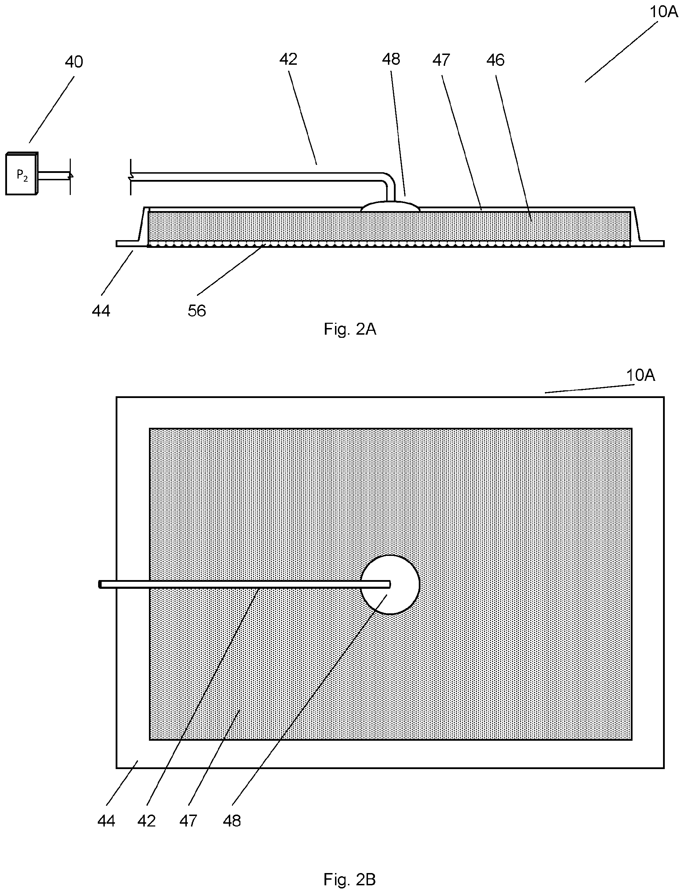

FIG. 2A depicts a cross-sectional side view of an embodiment of the NPWT wound care device 10A. Device 10A includes reticulated open cell foam layer 46, a film or other air impermeable material (air-sealed layer 47) over the upper surface of the foam layer 46, a fluid-permeable, non-adherent wound contact layer 56 on the lower surface of foam layer 46 opposite of air-sealed layer 47 which is configured to be placed in direct contact with open wound 36. Device 10A also includes a skin-safe adhesive deposited along the exterior perimeter 44 that is configured to form an air-seal against the patient's skin in conjunction with air-sealed layer 47. Device 10A also includes a negative pressure connector 48 attached to air-sealed layer 47 which may be coupled to a negative pressure air tube 42 connected to a negative pressure pump 40 and fluid reservoir (not shown) for collecting fluid from the wound.

FIG. 2B depicts an aerial view of device 10A shown in FIG. 2A. Although device 10A is depicted as a rectangular shape, as will be recognized by those skilled in the art, device 10A can be sized and shaped to accommodate all different sizes and shapes of wounds. For example, device 10A may be circular, oval, ellipsoid, curvilinear, or the like.

The device 10A may be applied in over the patient's open abdomen, skin graft or other wound with the contact layer 56 directly against the patient's body and covering the open wound. The skin-safe adhesive exterior perimeter 44 and the air impermeable layer 47 form a seal with the patient's skin to enclose both foam layer and contact layer 56 against the patient. The pump 40 creates a vacuum pressure P2 less than atmospheric pressure P1. This negative pressure is then supplied to the foam layer 46 via the air tube 42 and negative pressure connector 48 to compress the foam 46 and affix the device 10A to the patient. The negative pressure helps draw wound drainage through the contact layer 56 and the porous foam 46 where the drainage is collected in the reservoir (not shown) via pump 40.

The fluid-permeable non-adherent skin contact layer 56 protects the bowel, skin grafts, fistulas, or any other delicate structures (e.g., delicate organs) under device 10A, while allowing Negative Pressure Wound Therapy to control wound drainage and facilitate healing and the formation of granulating tissue.

In some examples, the NPWT may help protect and promote healing of the underlying wound. For example, the wound contact layer 56 may help to distribute the negative pressure and forces over the entirety of the open would while still promoting fluid drainage and closure of the wound. With conventional NPWT, an open cell foam is placed directly on an open wound. The negative pressure applied, in conjunction with the open cell structure of the foam, stimulates the underlying tissue to promote healing. This stimulation, while effective for certain types of tissue, may be too aggressive for delicate tissue (e.g., exposed bowels, organs, or viscera) and is contraindicated for such injuries. Including the wound contact layer 56 between the foam layer 46 and open wound of the patient may provide a convenient system that is minimally invasive and allows for the benefits of NPWT to be applied to such injuries without having the open cell foam directly contact such delicate tissue. Further, as the wound care device 10A may be provided as a unitary system, (e.g., as opposed to using separate and independent medical devices and articles) the device offers an all-in-one convenient system that helps reduce the complexity and duration of applying the wound care device to the patient.

In some embodiments, wound care device 10A may be provided as singular, prefabricated device wherein wound contact layer 56, foam layer 46, and air-sealed layer 47 are provided as a ready to use device that does not need to be assembled prior to use. Such a device 10A may significantly reduce the complexity and duration during wound care applications and changes, particularly in comparison to modulated systems that require multiple components to be sourced and applied (e.g., independent foam materials, independent adhesive drapes, and the like). In some examples, negative pressure connector 48, adhesive layer perimeter 44, and the like may also be provided as part of prefabricated device 10A or may be provided separate of device 10A or as part of a kit. In some such examples, a release liner may be applied to the adhesive layer and optionally the contact layer 56 to protect one or both layers during shipment. Additionally, wound care device 10A may be provided in a sterile pouch that can be opened prior to application to the patient.

The various layers and components of wound care device 10A may be construed using any suitable materials, films, foams, and the like including commercially available materials as will be recognized by those skilled in the art. For example, foam layer 46 may be formed from one or more biocompatible, sterilizable, materials including, for example, reticulated open cell foam used for medical purposes. In some examples, air-sealed layer 47 may be formed from one or more biocompatible, sterilizable, materials including, for example, adhesive plastic drape or film. Fluid-permeable contact layer 56 may be formed from one or more biocompatible, sterilizable, materials including, for example, non-adherent films, plastics, rubbers, silicones, polyester or other fabrics with pores, slits, porosities, or general permeability that allow the passage of fluid into the foam layer 46. In some examples, the contact layer 56 may itself be formed of a continuous layer of fluid permeable material such as commercially available materials Adaptic Touch, Mepitel One, Conformant 2, UrgoTul, Tegaderm, or the like. Additionally, or alternatively, the contact layer 56 may include perforations in the layer to allow for fluid to be drawn through the layer. In some embodiments, foam layer 46, contact layer 56, or both may contain antimicrobial materials, for example, silver or antibiotics.

In some embodiments, air-sealed layer 47 and wound contact layer 56 may be laminated together such that foam layer 46 is completely enclosed between the two layers. Additionally, or alternatively, air-sealed layer 47 may extend beyond the perimeter of foam layer 46 and or contact layer 56 such that air-sealed layer 47 forms the outer perimeter 44 of device 10A. The skin-safe adhesive may be deposited along this perimeter region to bond air-sealed layer 47 to a patient's skin, thereby enclosing the foam layer 46 and contact layer 56 underneath.

In some embodiments skin-safe adhesive exterior perimeter 44 may be formed from one or more biocompatible, sterilizable, materials including, for example, hydrocolloids, silicones, or adhesive plastic drapes or films. Depending on the perimeter construction of air-sealed layer 47 and contact layer 56, skin-safe adhesive may be deposited on air-sealed layer 47, contact layer 56, or both, to provide an air seal in conjunction with air-sealed layer 47 against a patient's skin.

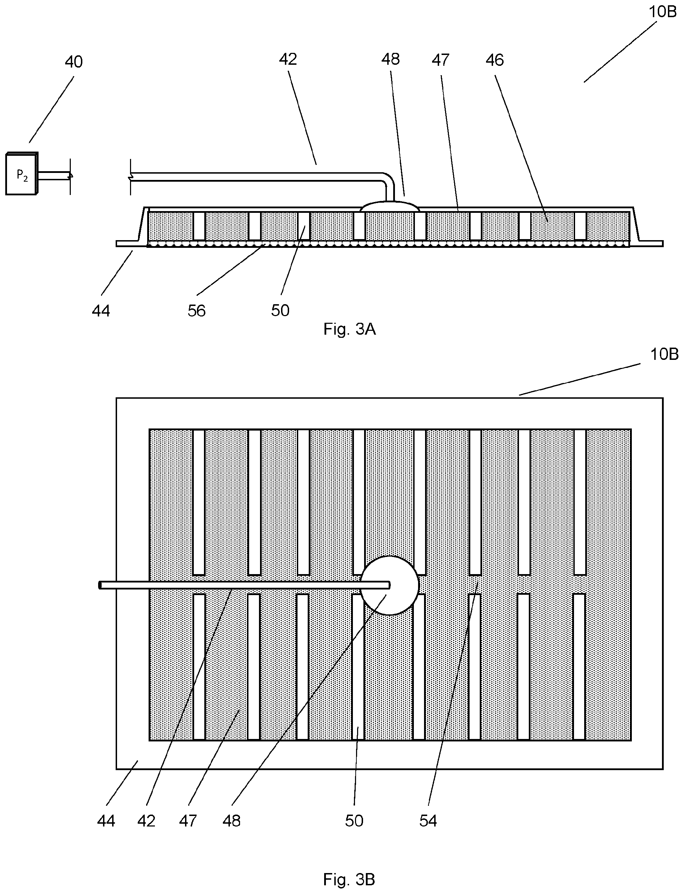

FIG. 3A depicts a cross-sectional side view of another example device 10B that includes channels 50 in the porous foam layer 46. FIG. 3B is an aerial view of the example device 10B shown in FIG. 3A further illustrating the channels 50. In some embodiments, channels 50 may help facilitate placement around non-planar (e.g., cylindrical) body surfaces and limbs. For example, the shape and size of channels 50 may be designed in a way that enables bending and flexibility along one or more axes. Foam bridges 54 within foam layer 56 may allow negative pressure communication through the entire device 10B. For example, foam bridges 54 may provide a continuous connection between sections of foam layer 56 such that the negative pressure draws air through the open cell structure and into a reservoir.

In some examples, the size and shape of channels 50 may be designed to help with the direction of lateral force (e.g., within the plane of device 10B) exerted by the device 10B during the NPWT. Such forces may be used to help draw the wound close as compared to an otherwise continuous sheet of foam.

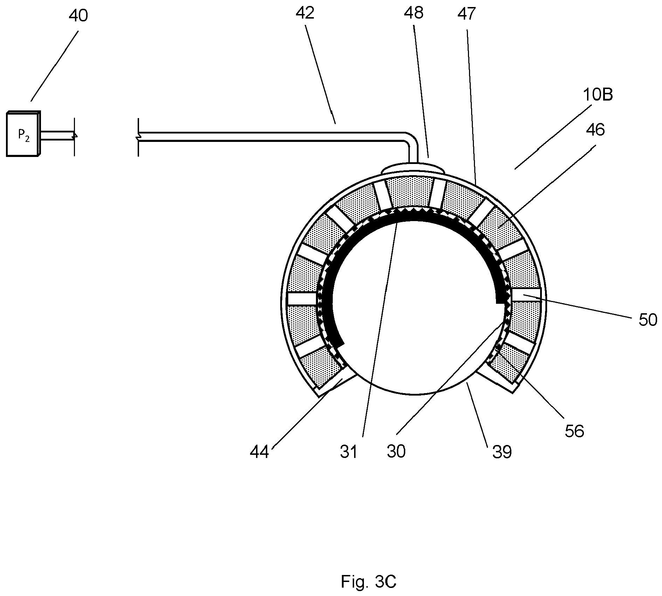

FIG. 3C is a cross-sectional side view of the example device 10B shown in FIG. 3A in an example employment to treat a wound 31 such as on a patient's limb or body 39. Device 10B is placed over the wound 31 and surrounding intact skin 30 where it is held in position by the skin-safe adhesive exterior perimeter 44. Negative pressure wound therapy is then supplied to the foam 46 via the air tube 42 and negative pressure connector 48 to compress the foam 46 and help affix the device 10B to the patient. The fluid-permeable non-adherent skin contact layer 56 protects the wound area and exposed delicate tissue under device 10B and allows drainage from the wound 31 to be drawn through the contact layer 56 and the porous foam 46 where the drainage is collected in a reservoir (not shown) connected to pump 40.

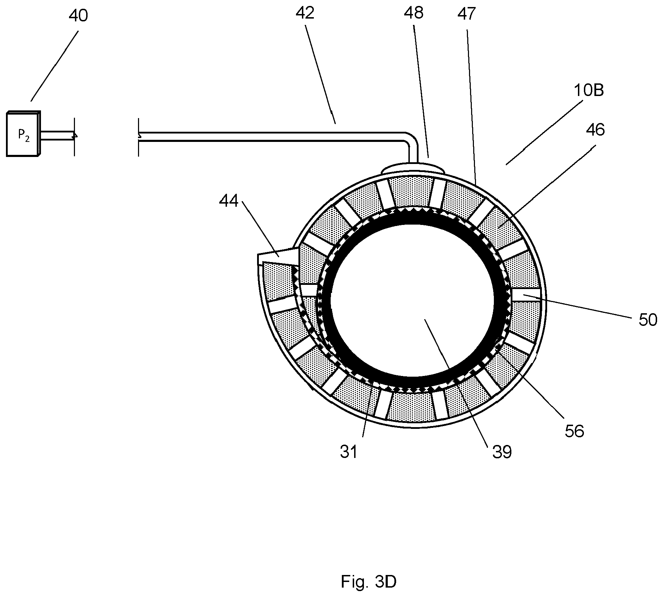

FIG. 3D is a cross-sectional side view of the example device 10B shown in FIG. 3A in an example employment to treat a circumferential wound 31 such as on a patient's limb or body 39. Device 10B is placed circumferentially around the limb or body 39 so that device 10B overlaps itself and covers the wound 31. Device 10B is attached to itself by fastening the adhesive exterior perimeter 44 on the top of the overlap to the air impermeable layer 47 on the bottom portion of the overlap. Negative pressure wound therapy is then supplied to the foam 46 via the air tube 42 and negative pressure connector 48 to compress the foam 46 and affix the overlapped device 10B around the patient's limb or body 39. Drainage from the wound 31 is drawn through the contact layer 56 and the porous foam 46 where the drainage is collected in a reservoir (not shown) connected to pump 40.

In such examples, wound care device 10B may be wrapped in a circumferential or spiral pattern around the limb or body of the patient to overlap with itself. A spirally-wrapped pattern may be of particular use when covering large open wounds such as those that span the entire length of a patient's leg. In some such examples, the width of device 10B need not be required to span the whole wound because perimeter edge 44 of the outer-wrapped layers of the device 10B will form an air-seal against the lower-wrapped air-sealed layer 47. Further, because the overlapping perimeter will form parts of the seal, portions of the lower-wrapped layers may be trimmed as needed. For example, portions of device 10B that would otherwise cause the adhesive perimeter 44 to directly contact the open wound may be trimmed away without sacrificing the seal integrity of the device 10B. Additionally, or alternatively, one skilled in the art will recognize the ability of multiple wound care 10 devices being used collectively together to cover a single large wound.

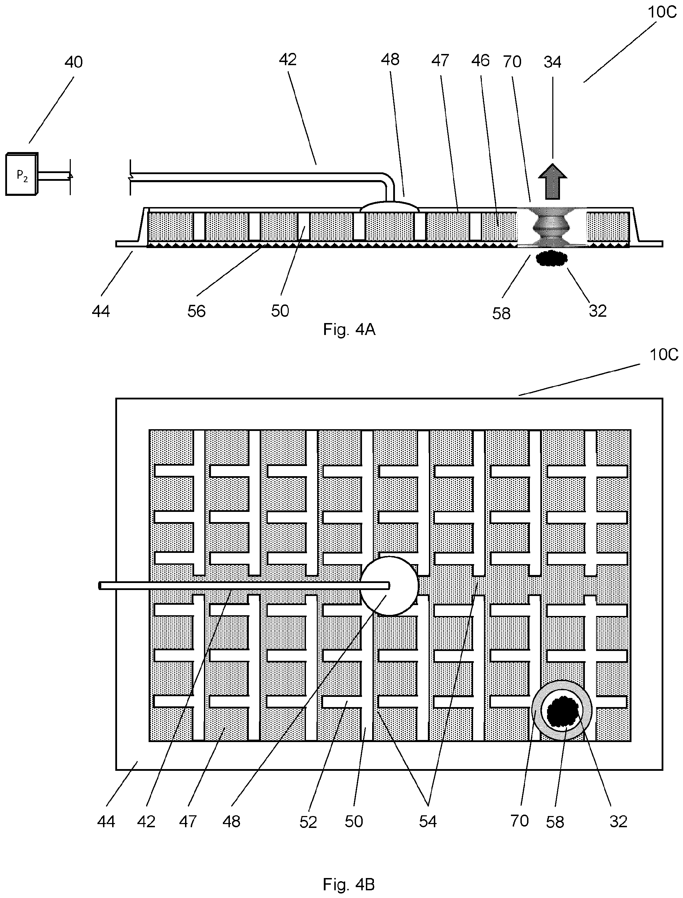

FIG. 4A depicts a cross-sectional side view of another example device 10C that incorporates an integral fistula and ostomy drainage isolator 70 placed in a way that directs the intestinal effluent 34 from an enteric fistula 32 away from the patient. FIG. 4B is an aerial view of the example device 10C shown in FIG. 4A which further illustrates the placement of the fistula and ostomy drainage isolator 70 around and enteric fistula 32. Also shown are channels 50 and cross channels 52 in the porous foam layer 46 to facilitate device 10C placement on uneven body surfaces.

The fistula and ostomy drainage isolator 70 may be embedded in the foam layer 46 and sealed to the air impermeable layer 47 and contact layer 56. An opening 58 through device 10C and drainage isolator 70 allows intestinal and other bodily fluid effluent 34 to pass through the drainage isolator 70 and away from the patient's body for collection into an ostomy pouch-type appliance. In some embodiments, drainage isolator 70 may be prefabricated within device 10C or inserted within the device 10C by the caregiver prior to patient application. For example, a caregiver may cut an opening through air-sealed layer 47, foam layer 46, and optionally contact layer 56. Drainage isolator 70 may then be inserted and sealed against air-sealed layer 47 to maintain the integrity of the negative pressure environment. A hole or cross slit may be placed within contact layer 56 to allow passage through opening 58 while also optionally providing a contact buffer layer between drainage isolator 70 and the wound surface.

In some embodiments, fistula and ostomy drainage isolator 70 may be formed from one or more biocompatible, sterilizable, materials including, for example, plastics or rubbers or silicone rubber. Other materials may be used, for example, a flexible thermoplastic. Preferably, fistula and ostomy drainage isolator 70 is formed using a non-fluid permeable and/or non-porous flexible material. Examples of such isolators 70 include the Fistula Funnel and Wound Crown available from KCI.

Further, as will be recognized by those skilled in the art, all parts of device 10C can be sized and shaped to accommodate all different sizes and shapes of wounds.

Device 10C also includes optional channels 50 and cross channels 52 within foam layer 46. These channels 50 and 52 may be designed in a way that enables bending and flexibility along two axes while also preserving optional foam bridges 54 that allow negative pressure communication through the entire device 10C.

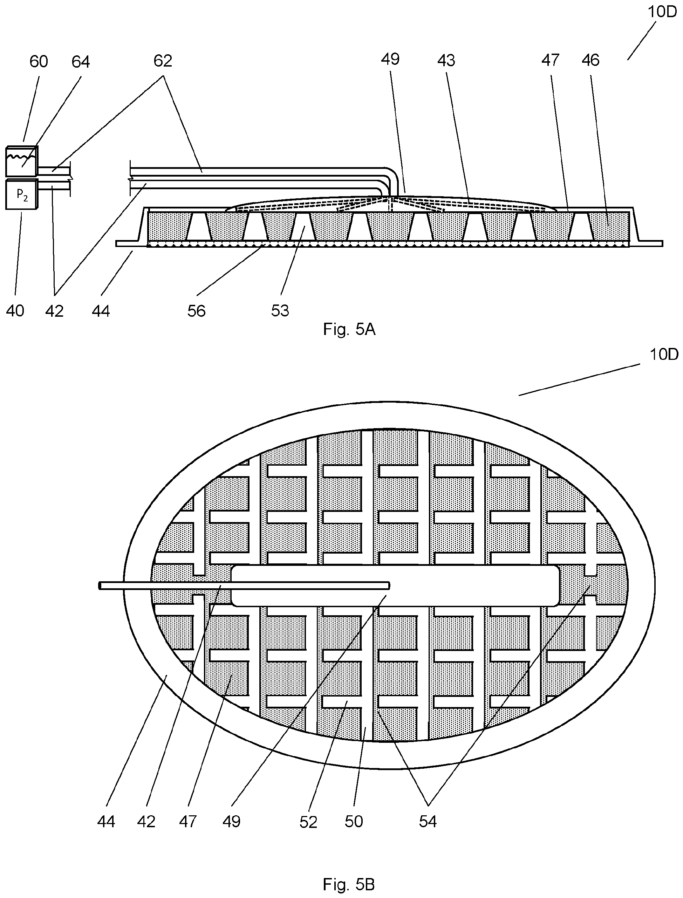

FIG. 5A depicts a cross-sectional side view of another example device 10D with alternative foam 46 shapes, a negative pressure manifold connector 49, and a wound soak fluid reservoir 60 and a wound soak fluid tube 62 for cleansing wounds. Device 10D is oval shape with angular shaped channels 53 in the reticulated open cell foam 46 to facilitate device 10D application on irregular body surfaces. Other device and channel shapes are also contemplated that may be appropriately sized and shaped to address particular types of injuries. The manifold connector 49 has multiple manifold channels 43 which are designed to evenly distribute negative pressure to multiple locations throughout the porous foam 46. The wound soak reservoir 60 holds saline solution or antimicrobial fluid 64 which can be pumped through the wound soak fluid tube 62 and negative pressure connector 49 to soak the foam 46 and cleanse the underlying wound. After cleansing, negative pressure supplied through tube 42 draws used soak fluid and wound exudates through the fluid-permeable non-adherent skin contact layer 56 and porous foam 46 into pump 40 where it is contained within a reservoir.

FIG. 5B is an aerial view of the example device 10D shown in FIG. 5A which further illustrates the oval shape of device 10D. Although this embodiment contemplates a rounded shape device 10D and shaped channels 53 in the reticulated open cell foam 46, as will be recognized by those skilled in the art, device 10D, shaped channels 53, and porous foam 46 can be sized and shaped to accommodate all different sizes and shapes of wounds.

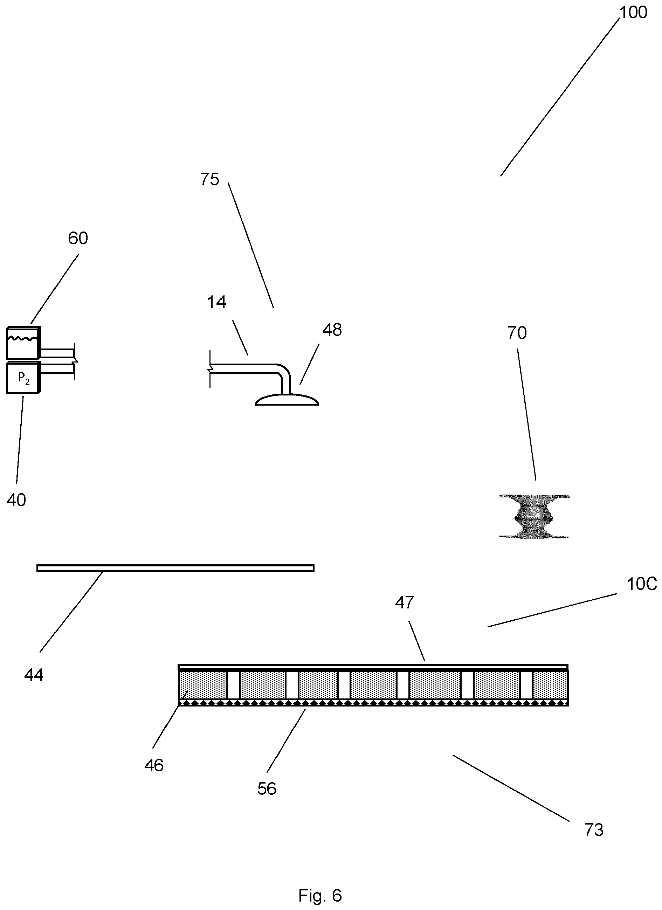

FIG. 6 is a view of an example medical kit 100 that includes wound care device 10C shown in FIG. 4A along with additional subcomponent options. Device 10C may be provided for use in whole or as subcomponents for assembly at the bedside as will be recognized by those skilled in the art. For example, porous foam layer 46, contact layer 56, and air impermeable layer 47 may be combined into a foam and contact layer subcomponent 73 which is cut to size at the bedside and tailored for the patients wound. The skin-safe adhesive exterior perimeter 44 may be provided separately and applied to the tailored foam and contact layer subcomponent 73 to create a seal with the patient's skin. Foam and contact layer subcomponent 73 may be selectively puncturable to allow placement of a fistula and ostomy drainage isolator 70 at any location in the device 10C and sealed in position with an air impermeable layer 47 or skin-safe adhesive exterior perimeter 44. Negative pressure connection subcomponent 75 can be fastened to foam and contact layer subcomponent 73 and connected to pump 40 with reservoir connected thereto. These or other subcomponents may be tailored and assembled at the time device 10C is placed on the patient.

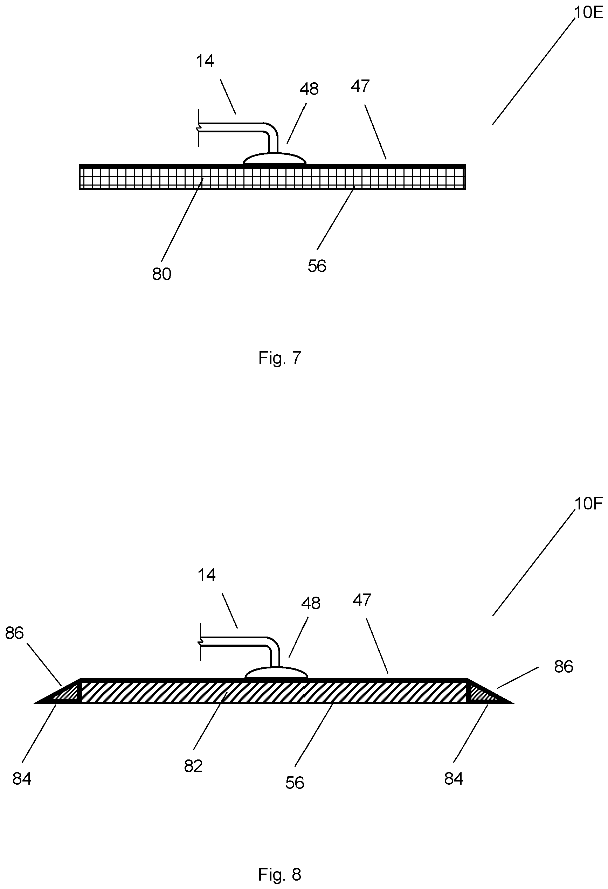

FIG. 7 is a cross sectional side view of another example device 10E showing an alternative construction with a unified porous contact layer 80 having an air impermeable upper layer 47 and fluid-permeable and non-adherent lower surface 56. Unified porous contact layer 80 may be fluid-permeable with open cells that allow negative pressure from the negative pressure connector 48 to communicate through the entirety of device 10E.

FIG. 8 is a side view of another example device 10F showing an alternative construction with a unified porous layer 82 having an air impermeable upper layer 47. Unified porous layer 82 may fluid-permeable and skin-safe with open cells that allow negative pressure from the negative pressure connector 48 to communicate through the entirety of device 10F. Porous layer 82 may also be configured to be self-sealing allowing the device 10F to be cut to a custom size or shape. Device 10F may be resized as need by cutting and compressing the upper and lower layers together to create a self-sealing perimeter region 84 when dressing is being placed to form an air impermeable seal 86 around the perimeter of device 10F. For example, an adhesive or adhesive layer may be disposed along the interior surface of air-sealed layer 47 that can be compressed porous layer 82 with towards the wound contact surface and/or to establish an air seal allowing device 10F to be cut to size. In some such embodiments, the adhesive layer may be protected by a liner that is removed upon cutting the device to size but otherwise would remain with the device if such a seal is not desired.

In some examples, the disclosed devices 10 may be sterilized for use in a surgical environment. Additionally, or alternatively device 10 subcomponents may be a provided as a kit that includes negative pressure pump 40 which may be sterilized and packaged in a sealed pouch or container that preserves sterility and can be opened by the patient or clinician.

Various embodiments of systems, devices, and methods have been described herein. These embodiments are given only by way of example and are not intended to limit the scope of the claimed inventions. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the claimed inventions.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

## Claims

1. A medical wound care device comprising:
an open cell foam layer;
a wound contact surface that is fluid permeable and configured to be placed in direct contact with an open wound; and
an air-sealed layer adjacent to the open cell foam layer on an opposite side of the wound contact surface,
wherein the medical wound care device is configured to be coupled to a negative pressure source and a reservoir to exert negative pressure wound therapy on an open wound to draw fluid through the wound contact surface through the open cell foam layer and into the reservoir.

2. The medical wound care device of claim 1, wherein the open cell foam layer comprises an open-cell foam material and one or more open channels separating portions of the open-cell foam material.

3. The medical wound care device of claim 2, wherein the one or more open channels are configured to increase the bendability of the medical wound care device along one or more axes.

4. The medical wound care device of claim 2 or 3, wherein the open cell foam layer defines:
a first open channel configured to increase the bendability of the medical wound care device along a first axis; and
a second open channel configured to increase the bendability of the medical wound care device along a second axis different than the first axis.

5. The medical wound care device of any one of the preceding claims, wherein the medical wound care device is configured to be cut to a different size or shape to exert the negative pressure wound therapy, and optionally or preferably wherein the medical wound care device is configured to self-seal after being cut to a different size or shape.

6. The medical wound care device of any one of the preceding claims, wherein the medical wound care device is configured to be selectively punctured by a drainage device to provide negative pressure wound therapy around a body opening and drainage from the body opening through the drainage device.

7. The medical wound care device of any one of the preceding claims, wherein:-
(i) the wound contact surface is non-adherent; and/or
(ii) the wound contact surface comprises an antimicrobial material.

8. The medical wound care device of any one of the preceding claims, wherein the open cell foam layer defines the wound contact surface.

9. A medical kit comprising the medical wound care device of any one of the preceding claims.

10. The medical wound care device of any one of claims 1 to 8 or the medical kit of claim 9, further comprising a manifold comprising a negative pressure connector configured to couple a negative pressure source and withdraw air through the negative pressure connector from the open cell foam layer to produce the negative pressure wound therapy.

11. The medical wound care device of claim 10 or the medical kit of claim 10, wherein the manifold is configured to withdraw air through the negative pressure connector from a plurality of different locations on the open cell foam layer to produce the negative pressure wound therapy.

12. The medical wound care device of claim 10 or 11 or the medical kit of claim 10 or 11, wherein the manifold further comprises a wound soak connector configured to couple to a source containing a wound soaking fluid that is introduced into the open cell foam layer through the wound soak connector to clean a wound.

13. The medical kit of any one of claims 10 to 12, wherein the manifold is separate from the medical wound care device and configured to attach to the air-sealed layer.

14. The medical kit of any one of claims 9 to 13, further comprising at least one adhesive film configured to be applied to a perimeter of the medical wound care device to form an air seal between the air-sealed layer and a patient's skin.

15. The medical wound care device of any one of claims 1 to 8 or 10 to 12 or the medical kit of any one of claims 9 to 13, wherein the medical wound care device comprises an adhesive along a perimeter of the wound care device configured to form an air-seal to contribute to the creation of the negative pressure wound therapy, and optionally or preferably wherein the adhesive comprises a skin-safe adhesive configured to form an air-seal with a patient's skin to contribute to the creation of the negative pressure wound therapy.

16. The medical wound care device of claim 14 or 15, or the medical kit of claim 14 or 15, wherein the adhesive is configured to form an air tight seal when brought in contact with an exterior surface of the air-sealed layer.

17. The medical kit of any one of claims 9 to 16, further comprising a drainage device, wherein the medical wound care device is configured to be selectively punctured by the drainage device to provide negative pressure wound therapy around a body opening and drainage from the body opening through the drainage device.
